# EUROPEAN PATENT SPECIFICATION

(11) **EP 1 478 410 B1**
(45) Date of publication and mention of the grant of the patent: **06.12.2006**
(21) Application number: 03700300.1
(22) Date of filing: 30.01.2003
(51) Int. Cl.: A61L 9/12, A61L 9/01

(54) **AIR-FRESHENING DEVICE**
LUFTERFRISCHUNGSGERÄT
DISPOSITIF DESODORISANT

(30) Priority: 28.02.2002 EP 02004584
(43) Date of publication of application: 24.11.2004
(73) Proprietor: Givaudan SA, 1214 Vernier (CH)
(72) Inventor: BLONDEAU, Philippe, F-75019 Paris (FR); QUELLET, Christian, CH-2502 Biel (CH); DUMAS, Sandrine, M. Krikorian, F-95290 L'Isle Adam (FR); PICCI, Sebastien, F-68260 Kingersheim (FR)
(74) Representative: McStea, John Anthony
(86) International application number: PCT/CH2003/000076
(87) International publication number: WO 2003/072151

(56) References cited:
- EP-A- 0 093 262
- EP-A- 0 419 850
- EP-A- 0 520 547
- EP-A- 1 082 969
- WO-A-98/41244
- US-A- 4 917 301
- US-A- 5 518 790
- US-A- 5 716 000
- US-A- 5 788 155
- PATENT ABSTRACTS OF JAPAN vol. 1997, no. 07, 31 July 1997 (1997-07-31) & JP 09 075439 A (DAINIPPON PRINTING CO LTD), 25 March 1997 (1997-03-25)

## Description

The invention relates to a fragrance composition for the controlled release of volatile substances into the ambient air, and to air-freshening devices containing the same.

There is an increasing demand for fragrance release systems, which provide a pleasant, natural aroma during a long period of time, e.g. 4 to 6 weeks, without significant changes of the fragrance strength and/or fragrance character during usage.

Gel compositions to control the degree of fragrance release from air-freshening devices are known. One of the first gels has been made from seaweed extracts, e.g. carrageenan. However, these gels can hold only a relatively small concentration of fragrance oils and have poor fragrance-release characteristics. The European Patent Application No. 0 631 788 discloses a gel-like fragrance composition which comprises an aqueous gel comprising an aqueous fluid and a gelling agent incorporated in an oil-absorbable resin carrying a fragrant material. It is stated therein that it is possible to achieve a well-balanced volatile emission for a long period, independently of the perfume character. These aqueous gel compositions may contain up to 20% by weight of a fragrant material. In the last years, other gelling agents that are able to hold much higher concentrations of fragrance oils have been used. The U.S. Patent No. 5 643 866 discloses a crystal clear, self-supporting, highly concentrated gel, comprising fragrance oils, glycols and dibenzylidene sorbitol acetal (DBSA) as gelling agent. Said gels can be loaded up to 80% with fragrance oils. US Patent No. 5 780 527 discloses a rigid dry and transparent gel obtained by reacting, in the presence of a fragrance composition, a liquid polymeric material with a cross-linking agent.

Non-gel like fragrance compositions are widespread in combination with electrical heated vapour dispensing devices, or in combination with other means, e.g. a wick, to control the release of the fragrance. Typically, a solvent is used to support the controlled release of the fragrance. For example the European Patent Application No. 1 078 640 describes a single-phase aqueous air freshening composition comprising 30% to 60% glycol ethers used in an air freshener device comprising a wick.

Several devices for perfuming ambient air having a sheet of permeable polymeric material are described, for example in US-A-4917301, EP 0 093 262, US 5,788,155 and EP 1 082 969. The prior art is silent on the nature of the fragrance containing composition and the effect that this may have on the performance of fragrance release.

There remains a need to provide air-freshener systems that are both aesthetically pleasing in design and which give still improved fragrance release in terms of duration and fragrance quality. We however made the surprising discovery that a breathable film performs very well in combination with a composition comprising an aqueous phase, and a hydrophobic phase containing the fragrance, in delivering a quantity of fragrance into the ambient air in a controlled and sustained manner.

Therefore, in a first aspect, the invention provides an air-freshening device comprising a container having an open end, the container holding a composition comprising an aqueous phase, and a hydrophobic phase comprising a fragrance, and said open end is covered by a breathable film which permits of egress of the fragrance and water-vapour.

Surprisingly we found that the addition of water to a fragrance not only influences the life-cycle of the air-freshening device but also is important for the essentially continuous release of the fragrance. The air-freshener device permits of strong fragrance release and the character of the released fragrance does not substantially alter over a long period of time, e.g. 4 to 6 weeks. We have found that the present of water influences the impact and quality of the released fragrance and leads to higher evaporation profile measured by the weight lost of the product over a time period of use as is more specifically illustrated in the examples. The rate of evaporation and therefore the life-cycle of the air-freshening device may be varied depending on the ratio of water to fragrance. In a preferred embodiment, the weight ratio between water to fragrance is about 9:1 to about 1:9, more preferably about 7:3 to about 3:7, most preferably 4:1 to about 2:3.

The strength of a fragrance can be evaluated by the measurement of the odour value, or by sniffing. The odour value of a fragrance material is defined as the ratio of the vapour pressure and its mean odour threshold concentration. The odour threshold concentration can be measured by standard methods, for example described by ASTM E679-91. To calculate the odour value of a fragrance composition the vapour pressure and odour threshold concentration of the fragrance has to be determined. Because of the time-consuming and elaborate nature of methods to determine the odour value the analysis of the fragrance strength by sniffing is preferred, as is well known in the perfumery arts.

The character of a fragrance (the so-called «hedonic character») relies on the contribution of all components of a fragrance through evaporation. Highly volatile fragrance components (so-called «Top Notes») are responsible for the initial impression of a fragrance. Less volatile fragrance components (so-called «Middle Notes», also known as «Heart Notes», and so-called «Bottom Notes», also known as «Base Notes») are responsible for the long-lasting impression of a fragrance. A fragrance's character depends on the harmonisation of Top Note, Middle Note, and Bottom Note. Clearly, it is highly desired that the released fragrance character does not significantly change during use of an air-freshening device over a prolonged period of time. The air-freshener devices of the present invention may permit substantially the same ease of passage to all fragrance ingredients irrespective of their physicochemical properties such as volatility and thus leads to the balanced nature of the fragrance emitted from the device. Without wishing to be bound by any theory applicant believes that due to the addition of water to the fragrance the developed water-vapour in a headspace of the device acts as a carrier for the fragrance to pass through the breathable film irrespective of their volatility.

The term «breathable film» as used herein means a polymeric film that allows water-vapour, but not water in the liquid phase, to pass through it. There are two different classes of breathable films known namely micro-porous films and monolithic films.

Micro-porous films are such films with small pores that permit passage of water-vapour. Typically, they comprises micropores having pore sizes of e.g. down to about 10µm. The small size of the pores prevents the penetration of liquid phase water across the film, but the interconnected pore structure allows the transmission of water-vapour. Micro-porous films may be made from, e.g. polyolefins, polytetrafluoroethylenes (PTFE) and precipitated polyurethanes.

Monolithic films used in the present invention are based on copolymers having so called «hard» and «soft» segments. The hard segments may be polyesters, for example glycol, propanediol or butanediol units and dicarboxylic acid units connected by ester functional groups; polyurethanes; or polyamides, for example polyamid 6 (PA-6), polyamid 11 (PA-11), or polyamid 12 (PA-12). The soft segments, responsible for the water-vapour transmission, may be formed by polyether units, for example polyethylene glycol (PEG), polypropylene glycol (PPG) or polytetramethylene glycol (PTMG) or mixtures thereof, and dicarboxylic acids, such as, for example terephtalic acid. Monolithic films based on polyester block hard segments are described in US Patents No. 5 800 928, 4 725 481, and 6 001 464;Monolithic films based on polyurethane hard segments are described in US Patents No. 5 800 928 and 6 001 464; Monolithic films based on polyamide hard segments are described in US Patents No. 5 800 928 and 5506 024.

Parameters including molecular weight of the soft segment and the glass transition temperature influence the flexibility of the film. Therefore, the molecular weight of the soft segment is preferably between 600 and 4000 g/mol. The glass transition temperature is preferably below 0°C. The amount of the soft segment may vary widely depending on the particular properties desired. However, in a preferred embodiment the amount of the soft segment may be between 15% and 40% by weight of the film, preferably between 20% and 30% by weight. The soft segment formed by polypropylene glycol is preferred. Most preferred are soft segments formed by polyethylene glycol, or a mixture of polyethylene glycol and polypropylene glycol.

Examples of monolithic breathable films are poly(ether-co-amide), e.g. PEBAX^{®} by Atofina and poly(ether-co-ester), e.g. HYTREL^{®} by DuPont.

Properties such as the hydrophilic nature of the film, its crystallinity and the presence and amount of certain additives, e.g. filler, may affect the breathability of the film. By «breathability» is meant the water-vapour transmission rate (WVT) or water-vapour permeability (WVP). Its measuring unit is g/m²/24h and it is calculated by measuring how many grammes of water-vapour pass through one square meter of film in 24 hours. A variety of test methods have been developed to measure this property, for example ASTM E96 and DIN 53 122. Although most standard methods are of similar concept there are variables, e.g. temperature and relative humidity of the test environment, the principle of the test method, i.e. upright dish water method, upright dish desiccant method, inverted cup method, and inverted water method, that have an influence on the calculated water-vapour transmission rate. Consequently, the results from different methods are not directly comparable.

The breathable film according to the present invention preferably may have a thickness from about 10 µm to about 50µm, more preferred from 15µm to about 40µm.

The container according to the present invention may be made of any material that is commonly used for air-freshener devices, e.g. glass or plastic. Such materials are preferred, which are stable in the presence of fragrance materials, e.g. essential oils. The container may be of any shape or configuration subject to it having an open end and capable of receiving aqueous and oil phases. Preferably, in the filled container the upper liquid surface does not abut the surface of the breathable film. Rather, in a preferred embodiment there should be a space between the upper surface of the liquid phase and the lower surface of the breathable film in which a head-space of fragrance and water-vapour may develop. This space is preferably not greater than 8cm, more preferably not greater than 3cm, for example between 1 cm and 2cm, most preferably not greater than 1 cm.

The breathable film may be attached to the container in a sealed relationship utilising any conventional means, such as an adhesive, heat seal or the like. Such means are preferred which form a strong bond between the film and the container to avoid the leakage of the liquid composition.

The hydrophobic phase according to the present invention consists of fragrance. A fragrance according to the present invention is a mixture of fragrance materials selected from such classes as acids, esters, alcohols, aldehydes, ketones, lactones, nitriles, ethers, acetates, hydrocarbons, sulfur- nitrogen- and oxygen-containing heterocyclic, polycyclic and macrocyclic compounds, as well essential oils of natural or synthetic origin. Such fragrance materials are described, for example, in S. Arctander Perfume Flavors and Chemicals Vols. 1 and 2, Arctander, Montclair, NJ USA 1969. The fragrance optionally may comprise odourless liquids such as dipropylene glycol, propylene glycol, diethylphtalate, benzyl benzoate, triethylcitrate, isopropylmyristate, carbitol, and hexylene glycol, or mixtures thereof. The appropriate choice of the liquid not only depends on the amount in which it may be used but also on the nature of the fragrance as will be fully appreciated by the person skilled in the art. Preferably, the fragrance comprises not more than 20% by weight of these liquids. A fragrance may contain residual water either due to the presence of a solvent or due to the production process. It is to be understood that in the context of the present invention water has to added addition to any residual water that may be present to form a composition according to the present invention. The composition according to the present invention comprises more than 10% by weight of water, more preferably more than 25% by weight, most preferably between 50% and 90%by weight of water. A skilled person will be aware that effects may also be achieved with low fragrance concentrations. Thus, a composition comprising 95% by weight of water or even more, e.g. 98%, may also be used to form a composition according to the present invention.

Optionally, the hydrophobic phase may comprise a lipophilic dye, for example Puricolor^{®} blue FBL5, Puricolor^{®} blue ABL9, Puricolor^{®} green U3 or Puricolor^{®} yellow AYE23 from Ciba.

For air-freshening devices fragrances are preferred comprising a high amount of fragrance material having a vapour pressure greater than 1000µg/l. These fragrance materials are classified as «Top Notes» and may be selected from acetate C6 hexylic, acetophenone, alcohol C6 hexylic, aldehyde C7 heptylic, aldehyde C9 isononylic, allyl caproate, allyl oenanthate, amyl butyrate, amyl vinyl carbinol, anapear, benzaldehyde, benzyl formate, benzyl methyl ether, bornyl acetate liquid, butyl acetate, camphene, carbitol, citronellal, cresyl methyl ether para, cyclal C, cymene para, decenal-4-trans, delta-3 carene, diethyl malonate, dihydro anethole, dihydro myrcenol, dimethyl octenone, dimetol, dimyrcetol, dipentene, estragole, ethyl acetate, ethyl acetoacetate, ethyl amyl ketone, ethyl benzoate, ethyl butyrate, ethyl caproate, ethyl isoamyl ketone, ethyl isobutyrate, ethyl methyl-2-butyrate, ethyl oenanthate, ethyl propionate, eucalyptol, fenchone alpha, fenchyl acetate, fenchyl alcohol, freskomenthe, geranodyle, guaiacol, hexenal-2-trans, hexenol-3-cis, hexenyl acetate, hexenyl-3-cis butyrate, hexenyl-3-cis formate, hexenyl-3-cis isobutyrate, hexenyl-3-cis methyl-2-butyrate, hexenyl-3-cis propionate, hexyl butyrate, hexyl isobutyrate, hexyl propionate, hydratropic aldehyde, isoamyl propionate, isobutyl isobutyrate, isocyclocitral, isopentyrate, isopropyl methyl-2-butyrate, isopulegol, leaf acetal, lime oxide, limetol, linalool oxide, linalool, manzanate, melonal, menthone, methyl amyl ketone, methyl benzoate, methyl camomille, methyl hexyl ketone, methyl pamplemousse, methyl salicylate, nonanyl acetate, ocimene, octenyl acetate, pandanol, pinene alpha, pinene beta, prenyl acetate, terpinene gamma, terpinolene, tetrahydro citral, tetrahydro linalool, tricyclal, and valerolactone gamma.

Fragrance materials having a vapour pressure between 10µg/l and 1000µg/l are classified as «Middle Notes» and may be selected from acetal E, acetal R, acetanisole, adoxal, agrumex, alcohol C10 decylic, alcohol C11 undecylenic, alcohol C12 lauric, alcohol C8 octylic, alcohol C9 nonylic, aldehyde C11 undecylenic, aldehyde C11 undecylic, aldehyde C12 lauric, aldehy, aldehyde iso C11, allyl amyl glycolate, allyl cyclohexyl propionate, ambrinol, amyl benzoate, amyl caproate, amyl cinnamic aldehyde, amyl phenyl acetate, amyl salicylate, anethole synthetic, anisyl acetate, anisyl alcohol, anther, aubepine para cresol, benzyl acetone, benzyl butyrate, benzyl isobutyrate, benzyl isovalerate, benzyl propionate, bergamyl acetate, berryflor, boisiris, butyl butyro lactate, butyl cyclohexanol para, butyl cyclohexyl acetate para, butyl quinoline secondary, carvone laevo, caryophyllene, cashmeran, cedrene epoxide, cedroxyde, cedryl methyl ether, celery ketone, centifolyl, cetonal, cetone alpha, cinnamic aldehyde, cinnamyl acetate, citral dimethyl acetal, citrodyle, citronellol, citronellyl acetate, citronellyl butyrate, citronellyl formate, citronellyl isobutyrate, citronellyl nitrile, citronellyl oxyacetaldehyde, citronellyl propionate, clonal, coniferan, creosol, cresyl acetate para, cresyl isobutyrate para, cumin nitrile, cuminic aldehyde, cuminyl alcohol, cyclamen aldehyde extra, cyclogalbanate, cyclohexyl ethyl acetate, cyclohexyl salicylate, cyclomethylene citronellol, cyperate, damascenone, decahydro naphthyl formate beta, decalactone delta, decalactone gamma, decatone, decyl methyl ether, delphone, dihexyl fumarate, dihydro ambrate, dihydro cyclacet, dihydro eugenol, dihydro farnesal, dihydro ionone beta, dihydro jasmone, dihydro linalool, dihydro terpineol, dimethyl anthranilate, dimethyl benzyl carbinol, dimethyl benzyl carbinyl acetate, dimethyl benzyl carbinyl butyrate, dimethyl phenyl ethyl carbinol, dimethyl phenyl ethyl carbinyl acetate, diphenyl methane, diphenyl oxide, dipropylene glycol, dupical, ebanol, ethyl caprylate, ethyl cinnamate, ethyl decadienoate, ethyl linalool, ethyl linalyl acetate, ethyl octenoate-2, ethyl pelargonate, ethyl phenoxy acetate, ethyl phenyl acetate, ethyl phenyl glycidate, ethyl salicylate, eugenol pure, eugenyl acetate, farnesene, fennaldehyde, fixambrene, floralozone, floramat, florol, floropal, folenox, folione, folrosia, fraistone, fructone, fruitate, gardenol, gardocyclene, georgywood, geraniol, geranitrile, geranitrile T, geranyl acetate, geranyl acetone, geranyl butyrate, geranyl crotonate, geranyl formate, geranyl isobutyrate, geranyl propionate, givescone, glycolierral, guaiyl acetate, gyrane, heliotropine crystals, hexenyl-3-cis benzoate, hexenyl-3-cis hexenoate, hexenyl-3-cis salicylate, hexenyl-3-cis tiglate, hexyl benzoate, hydroxycitronellal dimethyl acetal, indoflor, indole pure, indolene, ionone beta, irisantheme, irisone alpha, ironal, irone alpha, irone E, irone F, iso E super, isobornyl propionate, isobutyl benzoate, isobutyl phenyl acetate, isobutyl quinoline-2, isobutyl salicylate, isocaryol acetate, isoeugenol, jasmal, jasmin lactone delta, jasmin lactone gamma, jasmolactone, jasmone cis, jasmonyl, kephalis, kohinool, labienone, lactoscatone, lemarome N, lemonile, lierral, lilial, linalyl acetate, linalyl butyrate, linalyl formate, linalyl isobutyrate, linalyl propionate, lindenol, majantol, mayol, menthanyl acetate, metambrate, methoxy phenyl butanone, methyl acetophenone, methyl cinnamate, methyl cinnamic aldehyde, methyl decalactone gamma, methyl diantilis, methyl diphenyl ether, methyl ional beta, methyl isoeugenol, methyl octalactone, methyl octyl acetaldehyde, methyl octyne carbonate, methyl phenyl acetate, methyl quinoline para, moxalone, myraldene, neofolione, nerol C, neryl acetate, nonadyl, nopyl acetate, octahydro coumarin, octalactone delta, octalactone gamma, orcinyl 3, orivone, osyrol, oxyoctaline formate, parmavert, peach pure, pelargol, phenoxanol, phenoxy ethyl alcohol, phenoxy ethyl isobutyrate, phenyl ethyl acetate, phenyl ethyl alcohol, phenyl ethyl butyrate, phenyl ethyl formate, phenyl ethyl isobutyrate, phenyl propionic aldehyde, phenyl propyl acetate, phenyl propyl alcohol, pinoacetaldehyde, plicatone, precarone, prunolide, pyralone, radjanol, resedal, rhodinyl acetate, rhubafuran, rhubofix, rhuboflor, rosalva, sandalore, scentenal, skatole, spirambrene, stemone, strawberry pure, styrallyl propionate, syringa aldehyde, tangerinol, terpinene alpha, terpinyl acetate, terranil, tetrahydro linalyl acetate, tetrahydro myrcenol, tridecenonitrile, tropional, undecatriene, undecavertol, veloutone, verdol, verdyl acetate, verdyl propionate, vernaldehyde, vetynal, vetyvenal, and viridine.

Fragrance materials having a vapour pressure below 10µg/l are classified as «Bottom Notes» and may be selected from 2-benzyl-4-methanol-1,3-dioxolane, aldrone, ambrettolide, ambroxan, benzophenone, benzyl benzoate, benzyl cinnamate, benzyl phenyl acetate, cepionate, cetalox, citronellyl ethoxalate, civettone, cresyl caprylate para, cresyl phenyl acetate para, cyclohexal, diethyl phthalate, dione, dodecalactone delta, dodecalactone gamma, ethyl maltol, ethyl vanillin, ethylene brassylate, eugenyl phenyl acetate, evernyl, fixolide, florhydral, galaxolide, geranyl phenyl acetate, geranyl tiglate, grisalva, hedione, hexyl cinnamic aldehyde, hexyl salicylate, isomethyl cedryl ketone, laitone, linalyl benzoate, linalyl cinnamate, linalyl phenyl acetate, maltol, maltyl isobutyrate, methyl cedryl ketone, methyl dihydro isojasmonate, muscone, myraldyl acetate, nectaryl, okoumal, orange aldehyde, oranile, peonile, phenyl ethyl benzoate, phenyl ethyl cinnamate, phenyl ethyl phenyl acetate, propyl diantilis, rosacetol, rosaphen, sandela, thibetolide, timberol, triethyl citrate, undecalactone delta, vanillin, vanitrope, and velvione.

For air-freshening devices fragrances are preferred comprising from about 15% to about 80% by weight «Top Notes», more preferably from about 25% to about 60% by weight.

The aqueous phase according to the present invention may be water, for example distilled water. Other excipients optionally present in the aqueous phase are salts, for example sodium chloride and magnesium sulfate to minimize the migration of water soluble fragrance materials. Other excipients, e.g. water soluble dyes may also be present in the aqueous phase.

In order to prevent evaporation of fragrance during storage, the device may have fitted over the container and the breathable film, a non-breathable material, such as an aluminium foil, polyolefine films, ethylene acrylic acid copolymer films, polyethylene terephtalate films, or ethylen vinyl acetate films, which may be easily removed prior to use.

In a particular preferred embodiment of the present invention, the composition held in the container comprises an upper hydrophobic phase comprising a fragrance, and a lower aqueous phase.

In an alternative embodiment, the composition comprising the hydrophobic phase and the aqueous phase is provided as an emulsion.

The emulsion may optionally comprise a surfactant such as for example non-ionic surfactants, e.g. Neodol 91-8 from Shell Chemicals and Cremophor RH40 from BASF.

The invention has been described in terms of releasing fragrance. However, the device according to the invention may deliver other ingredients such as insect repellents, insect attractants, or materials having insecticidal activities, substance having antimicrobial activities, or mixtures thereof. In addition to these active principals, fragrance compositions may comprise excipients such as dyes and UV-absorbers.

For manufacturing a device according to the present invention the container may be filled in a first step with the aqueous phase followed by adding on top of the aqueous phase the hydrophobic phase. If the hydrophobic phase and the aqueous phase is provided as an emulsion, in a first step both phases may be prepared separately and then mixed together to form an emulsion, according to a process well known in the art, followed by filling the resulting emulsion into the container. After filling the container the open end of the container is covered by the breathable film, which itself might be covered by a removable impermeable film for storage.

While the invention has been illustrated and described with respect to an air-freshening device for the controlled release of volatile substances at room temperature, it will be apparent to those skilled in the art that the device may also find use in combination with electrical heated vapour dispensing devices.

The invention is illustrated by the following examples.

### Example 1 Preparation of different types of air-freshening devices and evaluation of there fragrance strength

A fragrance composition having citrus and spicy top notes with floral and middle notes and ambery and musky bottom notes was used in all examples.

### Comparative Product A (Liquid fragrance composition comprising a cellulose wick)

Blend under stirring at room temperature 8.0g fragrance and 8.0 g of Neodol (manufactured by Shell). Then, add 34g water, 25g dipropyleneglycolmethyleter, and 25g ethyl alcohol. Stir until the solution becomes clear. Pour 70 g of the solution into a glass container and insert a wick, made of cellulose in the middle of the container. The one end of the wick is in contact with the solution and the other end of it is in contact with the ambient air to supply the ambient air with fragrance.

### Comparative Product B (Solid gel fragrance composition comprising a gelling agent)

Blend at room temperature 1.735g Lithene N4-9000-10MA from Revertex Ltd with 8.0g fragrance together. Add under stirring 0.265g of Crodamet 02 from Revertex Ltd. Stir for about 10 minutes and pour 7g of the composition into a glass container.

### Product C (Liquid fragrance composition according to the present invention)

Pour 17.4g water into a glass container and add 5.6g fragrance. Seal the container with Pebax^{®} MV 6100 film from Atofina.

### Product A, B, and C were evaluated in booths.

The fragrance strength of each of the products was evaluated by an expert panel of 20 members by sniffing. The result was entered on a scale from 1 to 5, wherein 5 is very strong, 4 is strong, 3 is moderate, 2 is weak, and 1 is very weak. The average value is shown in Table 1.

**Table 1:**

| | Comp. Product A | Comp. Product B | Product C |
|---|---|---|---|
| Fresh product | 2 | 4 | 4.5 |
| After 1 week | 2 | 4 | 4 |
| After 2 weeks | 1 | 3 | 4 |
| After 4 weeks | 0.5 | 2 | 4 |

The fragrance strength of Product C is at all stages better than the fragrance strength of the comparison products, i.e. the wick system (Comp. Product A) permits at each age of the product only a weak egress of the fragrance and the gel system (Comp. Product B) releases a strong fragrance up to one week but the strength of the fragrance decreases over the period of time and was described to be weak after 4 weeks.

### Example 2: Evaluation of the fragrance character by headspace.

The ambient air in the booths of Product A, B, and C, prepared according to Example 1 were collected as fresh products, and 1, 2 and 4 weeks after preparation. Then the samples were analysed by GC/MS. The results are shown in Table 2. The principles of the headspace analysis is described in the Journal of Agriculture and Food Chemistry, Vol. 19, No. 6 (1971), page 1049-1056.

**Table 2:**

| | Fresh Product | After 1 week | After 2 weeks | After 4 weeks |
|---|---|---|---|---|
| | Comp. Product A | | | |
| Top Note | 95% | 87% | 82% | 77% |
| Middle Note | 4.7% | 12% | 16.2% | 21.4% |
| Bottom Note | 0.3% | 1% | 1.8% | 1.6% |

| | Comp. Product B | | | |
|---|---|---|---|---|
| Top Note | 95% | 74% | 68% | 72% |
| Middle Note | 4.6% | 24% | 30% | 25.3% |
| Bottom Note | 0.4% | 2% | 2% | 2.7% |

| | Product C | | | |
|---|---|---|---|---|
| Top Note | 93.5% | 93% | 92% | 91% |
| Middle Note | 6% | 6% | 6.5% | 6.2% |
| Bottom Note | 0.5% | 1% | 1.5% | 1.8% |

The concentration of the «Top Notes» decreases markedly for the comparative products, i.e. the wick system (Product A) and the gel system (Product B), whereas the concentration of the «Top Notes» remains very high for Product C during the evaluation period of 4 weeks.

### Example 3: Evaluation of the fragrance performance

Pour water into a glass container and add fragrance according to the mixture scheme given in Table 3 and seal the container sealed with Pebax^{®} MV 6100 film from Atofina.

**Table 3:**

| | Water (parts per weight) | Fragrance (parts per weight) | total |
|---|---|---|---|
| Mixture A | 0 | 100 | 100 |
| Mixture B | 30 | 70 | 100 |
| Mixture C | 50 | 50 | 100 |
| Mixture D | 80 | 20 | 100 |

Mixture A, B, C and D were evaluated by an expert panel in terms of strength and quality and resulted in the following ranking: Mixture D was better than Mixture C and Mixture C was better than Mixture B. The weakest was mixture A. If no water is added, Mixture A, the fragrance performance is very weak.

### Example 4: Evaluation of the weight lost

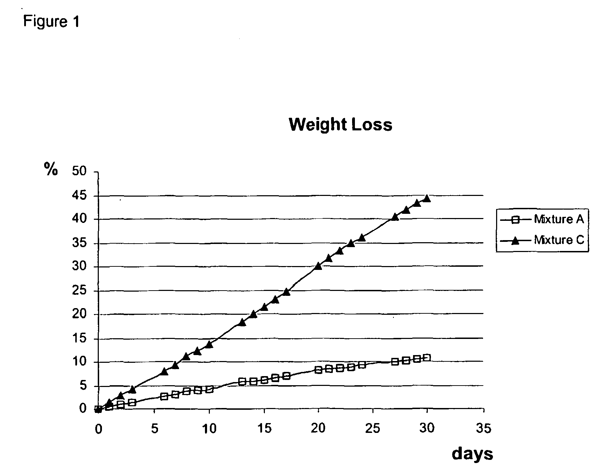

The weight lost of a glass container containing Mixture A (comparative product) and a container containing Mixture C from Example 3 have been measured on a daily base for a 30 day period. The results are shown in Figure 1.

## Claims

1. An air-freshening device comprising a container having an open end, the container containing a composition comprising an aqueous phase, and a hydrophobic phase comprising a fragrance, the composition comprising more than 10% by weight of water, **characterised in that** said open end of the container is covered by a breathable monolithic film.

2. An air-freshening device according to claim 1, the container containing a lower aqueous phase, and an upper hydrophobic phase comprising a fragrance.

3. An air-freshening device according to claim 1, the container containing an emulsion comprising an aqueous phase and a fragrance containing hydrophobic phase.

4. An air-freshening device according to claim 1 wherein the monolithic film is a copolymer comprising soft segments formed by polyether units selected from polyethylene glycol, polypropylene glycol or polytetramethylene glycol, or mixtures thereof.

5. An air-freshening device according to claim 4 wherein the monolithic film is selected from the group of poly(ether-co-amide) films and poly(ether-co-ester) films.

6. An air-freshening device according to any of the preceding claims, having a space between the upper surface of the composition and the lower surface of the breathable monolithic film.

7. An air-freshening device according to any of the preceding claims wherein a non-breathable material is releasably fitted over the breathable monolithic film.

8. A method of manufacturing an air-freshening device as defined in any of the preceding claims comprising the steps of
a) filling an aqueous phase in a container;
b) adding on top of the aqueous phase a hydrophobic phase comprising the fragrance so that a composition comprising more than 10% by weight of water is obtained; and
c) covering the open end of the container with a breathable monolithic film; and optionally
d) fitting a non-breathable film over the breathable monolithic film.

9. Us of an air-freshening device as defined in one of the claims 1 to 7 releasing fragrance to ambient air.

## Patentansprüche

1. Lufterfrischervorrichtung, umfassend einen Behälter, der ein offenes Ende aufweist, wobei der Behälter eine Zusammensetzung enthält, die eine wässrige Phase und eine hydrophobe Phase umfasst, wobei die hydrophobe Phase einen Duft umfasst, wobei die Zusammensetzung mehr als 10 Gew.-% Wasser umfasst, **dadurch gekennzeichnet, dass** das offene Ende des Behälters durch einen atmungsaktiven monolithischen Film bedeckt ist.

2. Lufterfrischervorrichtung nach Anspruch 1, wobei der Behälter eine untere wässrige Phase und eine obere hydrophobe Phase, die einen Duft umfasst, enthält.

3. Lufterfrischervorrichtung nach Anspruch 1, wobei der Behälter eine Emulsion enthält, die eine wässrige Phase und eine Duft enthaltende hydrophobe Phase umfasst.

4. Lufterfrischervorrichtung nach Anspruch 1, wobei der monolithische Film für ein Copolymer steht, das weiche Bereiche umfasst, die gebildet werden durch Polyethereinheiten, die ausgewählt sind aus Polyethylenglykol, Polypropylenglykol oder Polytetramethylenglykol oder Gemischen davon.

5. Lufterfrischervorrichtung nach Anspruch 4, wobei der monolithische Film ausgewählt ist aus der Gruppe von Polyethercoamidfilmen und Polyethercoesterfilmen.

6. Lufterfrischervorrichtung nach einem beliebigen der vorstehenden Ansprüche, die einen Raum zwischen der oberen Oberfläche der Zusammensetzung und der unteren Oberfläche des atmungsaktiven monolithischen Films aufweist.

7. Lufterfrischervorrichtung nach einem beliebigen der vorstehenden Ansprüche, wobei ein nicht atmungsaktives Material entfernbar über den atmungsaktiven monolithischen Film eingebaut ist.

8. Verfahren zur Herstellung einer Lufterfrischervorrichtung wie in einem beliebigen der vorstehenden Ansprüche definiert, umfassend die folgenden Stufen
a) Einfüllen einer wässrigen Phase in einen Behälter;
b) Zugeben einer hydrophoben Phase, die den Duft umfasst, auf die wässrige Phase oben, so dass eine Zusammensetzung erhalten wird, die mehr als 10 Gew.-% Wasser umfasst; und
c) Abdecken des offenen Endes des Behälters mit einem atmungsaktiven monolithischen Film; und gegebenenfalls
d) Einbauen eines nicht atmungsaktiven Films über den atmungsaktiven monolithischen Film.

9. Verwendung einer Lufterfrischervorrichtung wie in einem der Ansprüche 1 bis 7 definiert, die einen Duft an Umgebungsluft abgibt.

## Revendications

1. Dispositif de désodorisation de l'air, comprenant un récipient ayant une extrémité ouverte, le récipient contenant une composition comprenant une phase aqueuse, et une phase hydrophobe comprenant un parfum, la composition comprenant plus de 10 % en poids d'eau, **caractérisé en ce que** ladite extrémité ouverte du récipient est recouverte d'un film monolithique perméable à l'air.

2. Dispositif de désodorisation de l'air selon la revendication 1, le récipient contenant une phase aqueuse inférieure, et une phase hydrophobe supérieure comprenant un parfum.

3. Dispositif de désodorisation de l'air selon la revendication 1, le récipient contenant une émulsion comprenant une phase aqueuse et une phase hydrophobe contenant un parfum.

4. Dispositif de désodorisation de l'air selon la revendication 1, dans lequel le film monolithique est un copolymère comprenant des segments mous formés par des unités polyéther choisies parmi le polyéthylèneglycol, le polypropylèneglycol ou le polytétraméthylèneglycol, ou les mélanges de ceux-ci.

5. Dispositif de désodorisation de l'air selon la revendication 4, dans lequel le film monolithique est choisi dans l'ensemble consistant en les films de poly(éther-co-amide) et les films de poly(éther-co-ester).

6. Dispositif de désodorisation de l'air selon l'une quelconque des revendications précédentes, qui comporte un espace entre la surface supérieure de la composition et la surface inférieure du film monolithique perméable à l'air.

7. Dispositif de désodorisation de l'air selon l'une quelconque des revendications précédentes, dans lequel un matériau imperméable à l'air est disposé d'une manière amovible par-dessus le film monolithique perméable à l'air.

8. Procédé de fabrication d'un dispositif de désodorisation de l'air selon l'une quelconque des revendications précédentes, comprenant les étapes de
a) mise en place d'une phase aqueuse dans un récipient ;
b) addition, sur la partie supérieure de la phase aqueuse, d'une phase hydrophobe comprenant le parfum, de telle sorte que l'on obtienne une composition comprenant plus de 10 % en poids d'eau, et
c) la couverture de l'extrémité ouverte du récipient avec un film monolithique perméable à l'air ; et en option
d) la mise en place d'un film imperméable à l'air par-dessus le film monolithique perméable à l'air.

9. Utilisation d'un dispositif de désodorisation de l'air selon l'une quelconque des revendications 1 à 7, pour libération d'un parfum dans l'air ambiant.
